# EUROPÄISCHE PATENTSCHRIFT

(11) **EP 1 986 963 B1**
(45) Veröffentlichungstag und Bekanntmachung des Hinweises auf die Patenterteilung: **26.07.2017**
(21) Anmeldenummer: 06805488.1
(22) Anmeldetag: 23.10.2006
(51) Int. Cl.: C02F 11/04, B01J 8/00, C05F 17/02, C12M 1/107

(54) **VERFAHREN UND VORRICHTUNG ZUR KONTINUIERLICHEN VERFLÜSSIGUNG ORGANISCHER FESTSTOFFE**
PROCESS AND DEVICE FOR CONTINUOUS LIQUEFACTION OF ORGANIC SOLIDS
PROCÉDÉ ET DISPOSITIF POUR LIQUÉFIER EN CONTINU DES SUBSTANCES ORGANIQUES SOLIDES

(30) Priorität: 16.02.2006 DE 102006008026
(43) Veröffentlichungstag der Anmeldung: 05.11.2008
(73) Patentinhaber: Leibniz-Institut für Agrartechnik und Bioökonomie e.V. (ATB), 14469 Potsdam (DE)
(72) Erfinder: MUMME, Jan, 14469 Potsdam (DE); LINKE, Bernd, 14469 Potsdam (DE); TÖLLE, Rainer, 10115 Berlin (DE)
(74) Vertreter: Schubert, Klemens
(86) Internationale Anmeldenummer: PCT/DE2006/001891
(87) Internationale Veröffentlichungsnummer: WO 2007/093138

(56) Entgegenhaltungen:
- EP-A1- 0 033 017
- LINKE ET AL: "Ergebnisse aus den wissenschaftlichen Begleitungen der Pilotanlagen Pirow und Clausnitz" TROCKENFERMENTATION - STAND DER ENTWICKLUNGEN UND WEITERER F+E-BEDARF, Bd. 24, 4. Februar 2006 (2006-02-04), Seiten 112-130, XP009087961

## Beschreibung

Die Erfindung betrifft ein Verfahren und eine Vorrichtung zur kontinuierlichen Verflüssigung organischer Feststoffe sowie die Verwendung der Vorrichtung.

Organische Feststoffe werden fast ausschließlich in Biogasanlagen vergoren, die basierend auf dem Prinzip der vollständigen Durchmischung ursprünglich nur für die Behandlung von flüssigen Stoffen entwickelt wurden (Rührkessel-Fermenter). Durch den Einsatz von organischen Feststoffen in durchmischten Biogasanlagen ergeben sich erhebliche Nachteile für die Prozessführung und den Energiebedarf der Biogaserzeugung. So bergen der hohe Energiegehalt und das geringe Puffervermögen der festen Biomasse die latente Gefahr einer Überlastung und Störung des Biogasprozesses in sich. Um die Selbstentmischung der Feststoffe in der Gärflüssigkeit zu verhindern, müssen zudem hohe Energiemengen für die Homogenisierung durch Rührwerke oder Pumpen aufgewendet werden.

Mit dem Ziel, ein vorteilhaftes Verfahren für die Vergärung von Feststoffen bereitzustellen, wurden die so genannten Verfahren der Trockenvergärung entwickelt. Verfahren der Trockenvergärung lassen sich aufteilen in diskontinuierliche und kontinuierliche Betriebsweisen. Während diskontinuierliche Verfahren, die in Boxen, folienabgedeckten Mieten oder in Containern angewendet werden können, den Nachteil einer zeitlich stark schwankenden Produktivität besitzen, sind kontinuierliche Verfahren, zu denen der Gärkanal und Vertikalsilos eingesetzt werden, technisch sehr aufwendig zu realisieren.

In konventionellen Rührkessel-Fermentern gilt eine aufschwimmende Feststoffmasse als störend. Dieses basiert auf der Gefahr einer lokalen Prozessstörung durch Übersäuerung und Austrocknung sowie einer Behinderung des Biogasaustrages. Dem wird entgegen gewirkt durch intensives Homogenisieren mittels Pumpen oder Rührwerken.

EP 0 033 017 offenbart eine Vorrichtung und ein Verfahren zur Vergärung von Biomaterial wie Stroh und Mist. Die offenbarte Vorrichtung umfasst einen Reaktionsbehälter, der durch eine vertikale perforierte Trennwand in zwei Kammern unterteilt ist. Das zu vergärende Biomaterial wird in von unten in eine dieser beiden Kammer eingetragen, in der zweiten Kammer befinden sich Träger, auf welchen sich aktive Mikroorgansimen befinden. Durch die Perforation kann nur das wässrige Reaktionsmedium in die zweite Kammer übertreten, die Feststoffe werden zurückgehalten. Die festen Gärreste verlassen den Reaktionsbehälter, indem sie auf der Oberfläche der Einstauflüssigkeit aufschwimmen und in einen seitlichen Auslass übertreten.
Linke et al. beschreiben in ihrer Arbeit "Ergebnisse aus den wissenschaftlichen Begleitungen der Pilotanlagen Pirow und Clausnitz" , Trockenfermentation - Stand der Entwicklungen und weiterer F+E-Bedarf, Vol. 24, 4. Februar 2006, Verfahren zur Feststoffvergärung, die in den genannten Pilotanlagen durchgeführt wurden. Es werden Probleme diskutiert, die bei der Feststoffvergärung auftreten und Optimierungsversuche anhand der Ausbeuten an Biogas bewertet.

Aufgabe der vorliegenden Erfindung ist es eine einfache anlagen- und verfahrenstechnische Realisierung der kontinuierlichen Verflüssigung organischer Feststoffe zur Verfügung zu stellen.
Die Aufgabe der vorliegenden Erfindung wird durch ein Verfahren zur kontinuierlichen Verflüssigung organischer Feststoffe in einem Fermenter gelöst, bei dem man eine aufwärtsgerichtete Strömung der Feststoffe in einer Einstauflüssigkeit erzeugt, wobei man die Feststoffe im unteren Bereich des Fermenters zugibt, die festen Gärreste unterhalb des Einstauflüssigkeitsniveaus mittels zumindest eines Siebes im oberen Bereich des Fermenters sammelt, dadurch im Fermenter eine Pfropfenströmung fester zu vergärender organischer Substanzen mit einem nach oben ansteigenden Dichtegradienten erzeugt, die Einstauflüssigkeit durch den Fermenter zirkuliert, indem man sie durch den Flüssigkeitseinlass im unteren Bereich des Fermenters einbringt und durch die Flüssigkeitsentnahme im oberen Bereich des Fermenters ablässt, wobei man durch die Zirkulation der Einstauflüssigkeit im Fermenter eine aufwärtsgerichtete Strömung erzeugt, die das Aufschwimmen der Feststoffe unterstützt, die organischen Feststoffe für eine bestimmte Dauer in der Einstauflüssigkeit verweilen lässt, die festen Gärreste anschließend im oberen Bereich des Fermenters entnimmt, die Fermentationsgase im oberen Bereich des Fermenters entnimmt und die gelösten organischen Substanzen entnimmt.

Bei der Einstauflüssigkeit handelt es sich im Wesentlichen um Wasser, das im Laufe des Trockenvergärungsprozesses mit Reststoffen der Vergärung, wie zum Beispiel Zellsaft, angereichert wird.

Bei den Fermentationsgasen handelt es sich im Wesentlichen um Kohlendioxid, Methan, Wasserstoff und Schwefelwasserstoff. Sauerstoff wird, wenn er in den Fermenter gelangt, zügig verbraucht.

Als organische Feststoffe werden dem Fermenter zum Beispiel Bioabfälle, Grünschnitt, Gewerbeabfälle, Lebensmittelabfälle, landwirtschaftliche Abfälle, Küchenabfälle, organische Abfälle oder nachwachsende Rohstoffe und ähnliche Stoffe zugeführt.

Die festen Gärreste werden im oberen Bereich des Fermenters gesammelt. Das Sammeln erfolgt durch Siebe die unterhalb des Einstauflüssigkeitsniveaus angebracht sind oder sich zumindest in geringem Abstand über dem Einstauflüssigkeitsniveau befinden. Die Siebe sind dabei trichterförmig ausgeprägt, so dass eine Zuführung der festen Gärreste zur Gärresteentnahme bewirkt wird.

Die Erfindung vereint auf einfache und energetisch günstige Weise die Vorteile einer kontinuierlichen Betriebsweise mit den Vorteilen der Trockenvergärung.

Bei dem Verfahren der vorliegenden Erfindung handelt es sich um ein kontinuierliches Trockenvergärungsverfahren. Während bei einer nassen Vergärung der Fermenterinhalt in der Regel einen Wassergehalt von mehr als 90 % besitzt, arbeiten Trockenvergärungsverfahren mit einem durchschnittlichen Trockensubstanzgehalt im Fermenter oberhalb von 10 %. Stapelfähige Biomassen können in einem Nassfermenter vergoren werden. Entscheidend ist hierbei der Wassergehalt im Fermenter.

Die Prozessführung eines diskontinuierlichen Verfahrens und eines kontinuierlichen Verfahrens unterscheiden sich im Wesentlichen dadurch, dass bei diskontinuierlichen Verfahren die Zufuhr der Biomasse im Batch-Verfahren erfolgt und beim kontinuierlichen Verfahren eine Zufuhr der Biomasse im Durchfluss realisiert wird. Diese kontinuierliche Betriebsweise hat den Vorteil, dass eine gleichmäßige Produktivität erzielt wird.

Weitere Vorteile des erfindungsgemäßen Verfahrens sind die hohe Produktivität und Stabilität des Gärprozesses durch die hohen Konzentrationen abbaubarer Feststoffe und fester Puffersubstanzen im Fermenter und die einfache kompakte bauliche Gestaltung des Fermenters.

Das Verfahren der vorliegenden Erfindung ermöglicht es, die kontinuierliche Trockenvergärung von Feststoffen zur Herstellung von energiereichen flüssigen und/oder gasförmigen Abbauprodukten mit geringem verfahrenstechnischen Aufwand zu gestalten. Dabei kann durch die Wahl der geeigneten Verfahrensparameter die Verweildauer der Biomasse eingestellt werden.

Die optimale Verweildauer der organischen Feststoffe ist abhängig vom Einsatzzweck des Fermenters, der Art der eingesetzten Feststoffe und den Reaktionsbedingungen im Fermenter. Die optimale Verweildauer für Feststoffe liegt ungefähr in einem Bereich von 3 bis 28 Tagen.

Mit dem Verfahren der vorliegenden Erfindung können organische Feststoffe in hoher Konzentration verflüssigt werden. Dies bedeutet, dass der Durchsatz der Feststoffvergärung erhöht wird. Bei bekannten durchmischten Nassvergärungsverfahren werden täglich ungefähr 2-4 kg organische Trockensubstanz pro Kubikmeter Fermenterraum durchgesetzt. Durch die kontinuierliche, undurchmischte Verflüssigung der Feststoffe nach dem erfindungsgemäßen Verfahren können Durchsätze von 10 kg/m³ pro Tag oder mehr eingestellt werden, also mehr als die doppelte Menge.

Die Grenze des Durchsatzes von Feststoffen bei normalen durchmischten Vergärungsverfahren wird dadurch erreicht, dass eine zu große Zugabe von Feststoffen zu einer Übersäuerung im Fermenter führt, wodurch eine starke Hemmung der am Prozess beteiligten Bakterien auftritt.

Ein Vorteil des erfindungsgemäßen Verfahrens ist, dass durch die Feststoffverflüssigung und dem dadurch ermöglichten Abtransport gelöster Abbauprodukte es nicht zu einer solchen Übersäuerung kommt und somit ein höherer Durchsatz erzielt werden kann.

Der Feststoff-Durchsatz wird im erfindungsgemäßen Verfahren nur durch anlagentechnische Gegebenheiten begrenzt, prinzipiell sind auch 100 kg/m³ pro Tag möglich. Der Umsatz, also die Verflüssigungsrate, ist abhängig von biologischen und chemischen Faktoren. Die potentiell erreichbare Verflüssigungsrate ist sehr stark von der Art der eingesetzten Feststoffe abhängig. Bezogen auf die Masse an organischer Trockensubstanz wird die Verflüssigungsrate im Trocken- und Nassfermenter sich nicht wesentlich voneinander unterscheiden. Der Vorteil ergibt sich durch die höhere Konzentration an Feststoffen im Fermenter. Mindestens 50 % höhere Trockensubstanz-Konzentrationen sind möglich.

Möchte man einen größeren Umsatz erzielen, kann man ein zweistufiges Verfahren betreibt, um die in der ersten Stufe gewonnenen Reaktionsprodukte (Einstauflüssigkeit, feste Gärreste, Fermentationsgase) in einem weiteren Fermenter gezielt in die gewünschten Endprodukte, zum Beispiel Biogas, umzusetzen.

Eine dauernde Durchmischung des Materials ist nicht nötig, da sich das erfindungsgemäße Verfahren die Tendenz der Biomasse zur Bildung von Schwimmschichten zu nutze macht. Das Verfahren nutzt dabei für den Transport der Feststoffe im Fermenter das natürlicher Weise auftretende Aufschwimmen von organischen Feststoffen. Das Aufschwimmen wird dabei hervorgerufen durch die Anlagerung von Fermentationsgasblasen an die Feststoffpartikel und wird als Flotation bezeichnet. Es entsteht eine aufwärtsgerichtete Pfropfenströmung mit einem nach oben ansteigenden Dichtegradienten. Dies hat den Vorteil, dass nur ein geringer Prozessenergiebedarf durch die fehlende Homogenisierung der festen Reaktorinhalte und Nutzung der natürlichen Feststoff-Flotation zur Feststoffbewegung besteht.

Im Gegensatz zu konventionellen Verfahren in Rührkessel-Fermentern wird bei dem erfindungsgemäßen Verfahren im Fermenter eine aufwärtsgerichtete Feststoffströmung erzeugt. Dadurch wird eine kontinuierliche Betriebsweise des Fermenters ermöglicht. Die Nutzung der spontanen Feststoff-Flotation stellt zudem eine energetisch günstige Transportmethode dar.

Die Lagerungsdichte der zu vergärenden Feststoffe ist abhängig von der Feststoffart und kann im Fermenter lokal Maximalwerte von bis zu 0,8 t/m³ erreichen. Grundsätzlich nimmt die Lagerungsdichte nach oben hin zu. Im unteren Bereich bildet sich eine nahezu feststofffreie Zone aus. Im Mittel wird eine Dichte von 0,3 bis 0,5 t/m³ erwartet.

Der Trockensubstanzgehalt des Fermenterinhaltes steigt nach oben hin an und kann einen Wert bis zu 50 Masse% erreichen. Im Mittel wird ein Trockensubstanzgehalt zwischen 10 und 20 Masse% erwartet.

Für den Fall, dass der Feststoff-Fermenter zur Methanproduktion verwendet werden soll, ist eine Prozesstemperatur im (Trocken-)Fermenter zwischen 35-42 °C oder 50-65 °C am günstigsten. In diesem Bereich haben die mesophilen bzw. thermophilen Methanbakterien ihren Präferenzbereich. Wenn der Feststoff-Fermenter in erster Linie nur zur Verflüssigung der Feststoffe genutzt werden soll, können auch Temperaturen von bis zu 95 °C eingestellt werden. In diesem Bereich besitzen hydrolytisch aktive extremophile (65-85 °C) und hyperthermophile (> 85 °C) ihre Temperaturoptima. Neben einer Beschleunigung des Abbauprozesses ermöglichen diese Temperaturen auch eine wirksame Hygienisierung der Feststoffe sowie eine sichere Inaktivierung von Unkrautsamen.

Der pH-Wert im Feststoff-Fermenter kann lokal zwischen 3,5 und 8,5 liegen. Der pH-Wert steigt dabei nach oben hin an. Wenn in dem Feststoff-Fermenter eine Methanbildung etabliert werden soll, ist es günstig, eine möglichst große Fermenterzone mit neutralem pH-Wert (6,5 bis 7,5) zu erzeugen. Für die Feststoffverflüssigung ohne Methanbildung sind pH-Werte zwischen 5,2 und 6,3 günstig.

Für die Methanbildung sind anaerobe Bedingungen erforderlich.

Das C/N-Verhältnis sollte für die Methanbildung zwischen 20:1 und 30:1 liegen, für die Feststoff-Verflüssigung zwischen 10:1 und 45:1.

Die Feststoffe bringt man bevorzugt unterhalb der Flüssigkeitsfüllhöhe des Fermenters ein. Bei dem erfindungsgemäßen Verfahren wird konstruktiv sichergestellt, dass sich die aufsteigenden Feststoffe im Wesentlichen unterhalb des Flüssigkeitsniveaus befinden. Dadurch werden ein Austrocknen und eine Störung des Abflusses von Fermentationsgasen verhindert. Die Zwischenprodukte des Abbaus bleiben mobil, wodurch eine lokale Prozessstörung durch Produkthemmung vermieden wird.

In dem Fermenter wird eine Pfropfenströmung fester zu vergärender organischer Substanzen erzeugt. Hierbei erfolgt die Zugabe neuer Feststoffe im unteren Bereich des Fermenters und die Entnahme der festen Gärreste im oberen Bereich. Die Feststoffzugabe in den Fermenter wird durch aktive Fördermittel realisiert.

In einer bevorzugten Ausführungsform der Erfindung bringt man die Feststoffe mit einer flüssigkeitsdichten Fördereinrichtung in den Fermenter ein. Hierbei werden die Feststoffe aus mit einer flüssigkeitsdichten Förderschnecke aus einem Substratspeicher durch einen Kanal der horizontal durch die Fermenterwand verläuft in den Fermenter eingebracht.

Die Feststoffzugabe kann aber auch mit nicht flüssigkeitsdichten Fördereinrichtungen erfolgen.

In einer weiteren bevorzugten Ausführungsform der Erfindung bringt man die Feststoffe mit einer Fördereinrichtung in den Fermenter ein, welche eine Förderstrecke aufweist, die oberhalb des Flüssigkeitsniveaus des Fermenters beginnt. Hierzu bietet sich die Verwendung einer Förderschnecke an. Als Förderstrecke können bis zum unteren Fermenterbereich reichende Tauchrohre oder -schächte verwendet werden. Diese Rohre oder Schächte können sowohl innerhalb als auch außerhalb des Fermenters angeordnet sein.

In einer weiteren bevorzugten Ausführungsform bringt man die Feststoffe durch mechanische, hydraulische oder pneumatische Fördermethoden in den Fermenter ein. Die hydraulischen oder pneumatischen Fördermethoden können dabei alternativ oder unterstützend zur mechanischen Feststoffförderung eingesetzt werden. Als Transportmedium kann Einstauflüssigkeit und/oder Fermentationsgas verwendet werden.

In einer weiteren bevorzugten Ausführungsform der Erfindung verteilt man die Feststoffe im unteren Bereich des Fermenters. Das Verteilen der Feststoffe im unteren Bereich kann durch die unterschiedlichsten Verfahren erzeugt werde, so kann zum Beispiel eine Strömung durch das Einleiten der Einstauflüssigkeit oder der Fermentationsgase erzeugt werden. Eine weitere Möglichkeit ist die Verwendung eines Rührers.

Die festen Gärreste entnimmt man vorzugsweise mit einer Press- und/oder Förderschnecke. Dies geschieht im oberen Bereich des Fermeters zum Beispiel an der Decke des Fermenters. Die Press- und die Förderschnecke sind hierbei miteinander kombiniert.

Ferner kann die Press- und/oder Förderschnecke über eine Welle mit dem Rührer gekoppelt sein. Durch die Koppelung wird der Rührer nur langsam bewegt, da auch die Entnahme nur mit einer geringen Geschwindigkeit möglich ist. Durch die Koppelung kann somit ausgeschlossen werden, dass es zu einer Zerstörung der Feststoffschicht, durch zu starkes Verwirbeln der Feststoffe in der Einstauflüssigkeit, kommt.

Die Entnahme der festen Gärreste durch eine kombinierte Press- und Förderschnecke hat den Vorteil, dass die abgepresste Flüssigkeit, im Fermenter verbleibt. Die Gasdichtigkeit wird durch den sich in der Schnecke bildenden Feststoffpfropfen erreicht. Es sind jedoch auch andere Möglichkeiten zur Entnahme der festen Gärreste möglich.

In einer weiteren bevorzugten Ausführungsform der Erfindung leitet man die festen Gärreste mit einem Feststoffschaber in ein Fallrohr oder einen Schacht. Alternativ zu einer nach oben gerichteten Entnahme der festen Gärreste kann mittels eines Fallrohres oder -schachtes auch eine Abführung nach unten erfolgen. Diese Fallrohre oder -schächte können sowohl innerhalb als auch außerhalb des Fermenters angeordnet sein. Um die erforderliche Gasdichte zu erreichen, wird im unteren Bereich des Fallrohres oder Fallschachtes ein flüssigkeitsgefüllter Siphon oder eine Tauchung angebracht. Die Verlagerung der festen Gärreste in das Fallrohr oder in den Schacht kann durch einen rotierenden Feststoffschaber beschleunigt werden.

In noch einer weiteren bevorzugten Ausführungsform der Erfindung erzeugt man an der Position, an der die festen Gärreste entnommen werden, einen Unterduck. Durch das Anlegen eines Unterdrucks an Position der Feststoffentnahme kann der Auftrieb der Feststoffmasse erhöht werden. Hierdurch wird eine weitere Steigung des Dichtegradienten erreicht, wodurch die Abtrennung der festen und flüssigen Stoffphasen beschleunigt werden kann.

Die Fermentationsgase und die Einstauflüssigkeit trennt man vorzugsweise durch Sieben von den Feststoffen abtrennt. Dabei werden die Siebe gleichzeitig zum Niederhalten der Feststoffe genutzt. Die Abtrennung der Fermentationsgase kann durch die Einrichtung von weiteren Siebflächen im mittleren und unteren Fermenterbereich verbessert werden.

Die Einstauflüssigkeit, Teile der festen Gärreste und/oder Teile der Fermentationsgase zirkuliert man vorzugsweise durch den Fermenter oder durch mehrere Fermenter. Durch die Zirkulation der Einstauflüssigkeit und/oder der Fermentationsgase können diese beispielsweise als hydraulische oder pneumatische Fördermittel eingesetzt werden. Auch ist es möglich die Einstauflüssigkeit und/oder die Feststoffe in einen weitern Fermenter zu überführen. Von diesen zweiten Fermenter können die Einstauflüssigkeit und/oder die Feststoffe wieder in den ersten Fermenter zurückgeführt werden oder dem Kreislauf entzogen werden.

Zur weiteren Erhöhung der Prozessleistung und Stabilität der Fermentationsgasbildung können neben der Einstauflüssigkeit auch feste Gärreste in den Fermenter rückgeführt werden. Die Erhöhung der Prozessleistung und -stabilität basiert dabei hauptsächlich auf der Rückführung der benötigten Mikroorganismen sowie säurepuffernder Substanzen.

Bevorzugt überführt man die Einstauflüssigkeit aus dem Fermenter in einen zweiten Fermenter und von dort wieder zurück in den ersten Fermenter. Durch die Kombination des Fermenters mit einem separaten Methanisierungsreaktor zur Behandlung der Einstauflüssigkeit kann eine weitere Erhöhung der Prozessstabilität und Leistungsfähigkeit ermöglicht werden. Hierzu wird die Einstauflüssigkeit vom Fermenter in den Methanisierungsreaktor und von dort wieder in den Fermenter geleitet.

Die Einstauflüssigkeit und/oder die festen Gärreste überführt man vorzugsweise aus dem Fermenter in einem zweiten Fermenter, wobei man in dem zweiten Fermenter ein methan- und/oder wasserstoffhaltiges Fermentationsgas erzeugt, das man bei Bedarf in den ersten Fermenter zurückführt. Zur Erhöhung des Durchsatzes werden in dem ersten Fermenter Vorstufen der Biogasherstellung hergestellt, die dann in einem zweiten Fermenter zu Biogas umgesetzt werden. Hierzu wird in dem zweiten Fermenter der Anteil des Methans in dem Fermentationsgas erhöht.

Das auf diese Weise im zweiten Fermenter hergestellt Fermentationsgas.kann entweder dem Prozess entnommen werden oder wieder in den ersten Fermenter überführt werden.

Die festen Gärreste entnimmt man vorzugsweise dem Fermenter und überführt sie wieder in den Fermenter oder leitet sie aus dem Fermenter in einen zweiten Fermenter. Die festen Gärreste können dabei direkt in denselben Fermenter zurückgeführt werden. Auf diese Weise können die festen Gärreste wieder als Feststoffe in den Fermenter eingebracht werden. Eine weitere Möglichkeit besteht darin, die festen Gärreste in einem zweiten Fermenter nachzubehandeln. Die zurückgeführten Feststoffe können aber auch zur Erstbeimpfung eines Trocken-Fermenters verwendet werden, wodurch die Anlaufphase des Fermentationsprozesses beschleunigt werden kann.

Bevorzugt bringt man Enzyme, Mikroorganismen und/oder Spurenelemente in den Fermenter ein. Durch einen solchen Einsatz von Enzymen oder Mikroorganismen ist eine weitere Prozessbeschleunigung möglich, da die organischen Feststoffe schneller abgebaut werden können.

Förderlich kann die Zugabe der folgende Substanzen sein: Luft, Fermentationsgase, Enzyme, Säurepuffer, Spurenelemente und/oder Mikroorganismen.

Luft kann die Feststoff-Verflüssigung beschleunigen, hemmt jedoch die Methanbildung.

Fermentationsgase dienen der Erhöhung des Feststoff-Auftriebs zum Aufwirbeln von Sedimentationsschlamm am Fermenterboden und zur Vergleichmäßigung des Feststoffgehaltes im unteren Fermenterbereich.

Als Enzyme können Cellulasen, Hemicellulasen, Lipasen, Proteasen und/oder Amylasen hinzugefügt werden.

Als Säurepuffer können zum Beispiel Puffer auf Kalk- oder Ammoniumbasis verwendet werden.

Als Spurenelemente können zum Beispiel Nickel, Cobalt, Molybdän, Selen und/oder Wolfram hinzugefügt werden. Insbesondere Methanbakterien haben einen hohen Bedarf an Spurenelementen.

Mikroorganismen können, insbesondere zur Beschleunigung der Anlaufphase, zum Beispiel in Form von flüssigem oder festem Impfmaterial aus anderen Fermentationsanlagen hinzugefügt werden.

Weiterhin wird die Aufgabe der vorliegenden Erfindung durch eine Verflüssigungsanlage gelöst, wobei der Fermenter im oberen Bereich mindestens eine Flüssigkeitsentnahme, mindestens ein Sieb, das unterhalb des Einstauflüssigkeitsniveaus angebracht ist, zum Sammeln der festen Gärreste im oberen Bereich des Fermenters, sowie
im unteren Bereich jeweils mindestens einen Feststoffeinlass und ein Flüssigkeitseinlass und
im oberen Bereich jeweils mindestens eine Gärrestentnahme und eine Gasentnahme enthält.
Im unteren Bereich des Fermenters werden die organischen Feststoffe mit der Einstauflüssigkeit versetzt. Durch den Gärprozess schwimmen die Feststoffe auf und werden durch ein Sieb aufgesammelt und im oberen Bereich des Fermenters ausgebracht. Durch das Sieb werden die festen Gärreste von den Fermentationsgasen und der Einstauflüssigkeit getrennt. Die Fermentationsgase und die Einstauflüssigkeit werden im oberen Bereich des Fermenters durch eine Gas- bzw. Flüssigkeitsentnahme aus den Fermenter abgeleitet. Es ist aber auch möglich, die Flüssigkeitsabführung an anderen Stellen vorzunehmen. Denkbar wäre zum Beispiel auch eine horizontale Flüssigkeitsströmung mit seitlich angebrachten Ein- und Auslässen.

Die dem Fermenter entzogenen Fermentationsgase können wieder zurück in den Fermenter geleitet werden. Der Fermenter weist daher vorzugsweise mindestens einen Gaseinlass auf. Es ist aber auch möglich frische Gase, die nicht aus Fermentationsprozessen stammen, in den Fermenter zu leiten. Hierdurch kann zum Beispiel die Durchmischung der Feststoffe und der Einstauflüssigkeit gefördert werden.

In einer bevorzugten Ausführungsform der Erfindung ist an dem Feststoffeinlass des Fermenters eine flüssigkeitsdichte Fördereinrichtung angeordnet. Hierdurch wird ein Einbringen der Feststoffe unterhalb der Flüssigkeitsfüllhöhe des Fermenters ermöglicht. Ferner ist es möglich den Feststoffgrad im Fermenter gezielt einzustellen.

In einer weiteren bevorzugten Ausführungsform der Erfindung ist an dem Feststoffeinlass des Fermenters eine Fördereinrichtung angebracht, wobei der Beginn der Förderstrecke oberhalb des Flüssigkeitsniveaus des Fermenters angeordnet ist. Bei dieser Ausführungsform kann zum Beispiel eine Förderschnecke zur Beschickung des Fermenters mit Feststoffen verwendet werden. Als Förderstrecke können dabei bis zum unteren Fermenterbereich reichende Tauchrohre oder -schächte verwendet werden, wobei diese Rohre oder Schächte sowohl innerhalb als auch außerhalb des Fermenters angeordnet sein können.

In noch einer weiteren Ausführungsform ist vorzugsweise an dem Feststoffeinlass des Fermenters eine Vorrichtung zur mechanischen, hydraulischen oder pneumatischen Förderung der Feststoffe angeordnet. Als Transportmedium kann die Einstauflüssigkeit beziehungsweise Fermentationsgase verwendet werden, die in einem Kreislauf durch den Fermenter gepumpt werden.

Vorzugsweise ist die Gärresteentnahme des Fermenters eine Press- und/oder Förderschnecke. Die Gärresteentnahme sollte im oberen Bereich des Fermenters, zum Beispiel zentral an der Decke des Fermenters, angebracht sein. Die abgepresste Einstauflüssigkeit verbleibt dabei im Fermenter. Die Gasdichtigkeit wird durch den sich in der Schnecke bildenden Feststoffpfropfen erreicht. Eine Optimierung der Gasdichtigkeit kann durch die Tauchung eines die Gärresteentnahme umgebenden Mantels erreicht werden.

In einer weiteren bevorzugten Ausführungsform der Erfindung ist die Gärresteentnahme des Fermenters in einem Fallrohr oder Fallschacht ausgebildet, wobei im unteren Bereich des Fallrohres oder Fallschachtes ein flüssigkeitsgefüllter Siphon oder eine Tauchung angebracht ist.

Die Gärresteentnahme des Fermenters weist bevorzugt einen rotierenden Feststoffschaber auf, der die festen Gärreste in das Fallrohr oder den Schacht befördert.

An der Gärresteentnahme des Fermenters ist vorzugsweise ein Unterdruck erzeugbar. Dieser Unterdruck kann durch Pumpen erzeugt werden. Durch den Unterdruck wird zudem die aufwärtsgerichtete Strömung der Feststoffe beschleunigt.

Auf dem Boden des Fermenters ist vorzugsweise ein Rührer angebracht. Auf diese Weise können die dem Fermenter neu zugeführten Feststoffe gleichmäßig verteilt werden. Die aufsteigende Feststoffschicht wird jedoch nicht mehr aufgerührt.

Weiter bevorzugt sind die Press- und/oder Förderschnecke der Gärresteentnahme des Fermenters und der Rührer miteinander gekoppelt. Durch die langsame Bewegung der Förderschnecke wird auch der Rührer nur mit einer geringen Geschwindigkeit bewegt, die gerade ausreichend ist, um die neu zugeführten Feststoffe und die Einstauflüssigkeit zu durchmischen, jedoch nicht zu einer Verwirbelung der bereits aufgestiegenen Feststoffe führt und auch das weitere Aufschwimmen der Feststoffe nicht beeinträchtigt.

Im oberen Bereich des Fermenters werden durch ein Sieb die Fermentationsgase und die Einstauflüssigkeit von den Feststoffen abgetrennt.

Das Sieb des Fermenters ist vorzugsweise trichterförmig. Hierdurch wird eine selbstständige Bewegung der Feststoffe beziehungsweise der festen Gärreste in Richtung der Gärresteentnahme erzeugt.

Das Sieb besteht aus schräg angeordneten Lammellen, die eine Verstopfung der Sieböffnung vermeiden.

Vorzugsweise sind weitere Siebflächen im mittleren und unteren Bereich des Fermenters angeordnet, wodurch eine verbesserte Abtrennung der Fermentationsgase erzielt wird.

Die Einstauflüssigkeit und/oder die festen Gärreste sind vorzugsweise vom Fermenter durch mindestens einen weiteren Fermenter zyklisierbar. Ein Vorteil einer separaten Behandlung der Einstauflüssigkeit in einen zweien Fermenter ist, dass dieser speziell für den Abbau gelöster Zwischenprodukte ausgelegt werden kann. Dies ermöglicht eine Reduzierung der Konzentration an organischen Säuren im Fermenter. Hierdurch wird der Gefahr einer Übersäuerung entgegengewirkt und es ist eine deutliche Erhöhung der Belastung des Fermenters mit organischen Feststoffen möglich. In dem speziell auf den Abbau gelöster organischer Stoffe ausgelegten zweiten Fermenter können zudem außerordentlich hohe Biogasbildungsraten erzielt werden. Wodurch die Leistungsfähigkeit des Gesamtsystems erheblich gesteigert werden kann. Geeignete Fermentertypen für den Abbau gelöster Stoffe sind unter anderem Festbettreaktoren und Schlammbettreaktoren.

Des Weiteren wird die Aufgabe der vorliegenden Erfindung durch eine Verwendung der Verflüssigungsanlage zur Herstellung eines methan- und/oder wasserstoffhaltigen Fermentationsgases gelöst. Abhängig von den in den Fermenter eingebrachten Feststoffen und den Reaktionsbedingungen kann die Zusammensetzung des Fermentationsgases bei seiner Herstellung gesteuert werden. So kann das Fermentationsgas einen erhöhten Anteil an Wasserstoff und/oder Methan enthalten.

Das Verfahren zur kontinuierlichen Verflüssigung organischer Feststoffe dient der Produktion von Vorprodukten für die Biogasproduktion. Es ist jedoch auch möglich, das Verfahren zur direkten Erzeugung von Biogas zu verwenden.

Ferner wird die Aufgabe der vorliegenden Erfindung durch eine Verwendung der Verflüssigungsanlage zur Herstellung von organischen Säuren, die man in mindestens einem weiteren Fermenter zu einem methan- und/oder wasserstoffhaltigen Fermentationsgas abbaut gelöst. Die organischen Säuren dienen als Vorstufen zur Herstellung von Fermentationsgasen mit einem erhöhten Anteil an Wasserstoff oder an Methan.

Weiterhin wird die Aufgabe der vorliegenden Erfindung durch eine Verwendung der Verflüssigungsanlage zur Entkeimung von Speiseresten gelöst.

Mit dem Verfahren kann auch eine Pasteurisierung von Speiseabfällen durchgeführt werden.

Im Folgenden wird die Erfindung anhand von Zeichnungen näher erläutert. Im Einzelnen zeigt
- Figur 1: einen Querschnitt durch einen Fermenter,
- Figur 2: einen Querschnitt durch einen Fermenter mit Rührer,
- Figur 3: einen Querschnitt durch einen Fermenter mit Gaszuführung am Fermenterboden,
- Figur 4: einen Querschnitt durch den Fermenter mit einer Feststoff-Zuführung durch ein außen liegendes Tauchrohr,
- Figur 5: einen Querschnitt durch die Gärresteentnahme
- Figur 6: eine schematische Darstellung einer Verflüssigungsanlage.

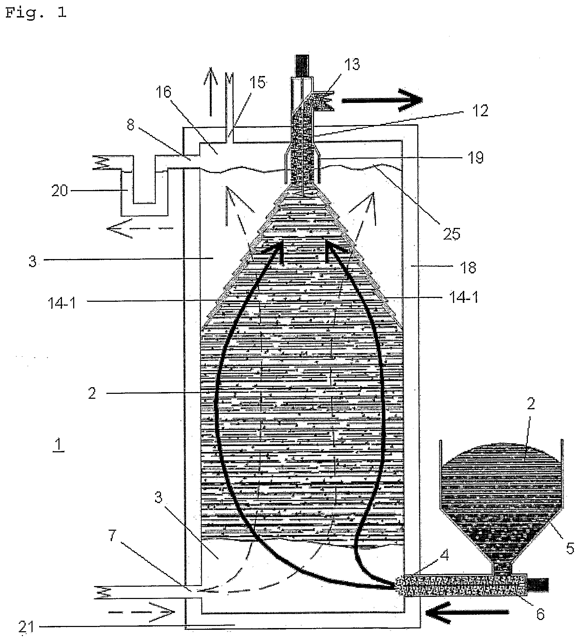

Die Figur 1 zeigt einen Querschnitt durch einen Fermenter 1 zur kontinuierlichen Verflüssigung organischer Feststoffe 2. Der Strom der Feststoffe 2 durch den Fermenter 1 ist in der Figur 1 durch dicke Pfeile, der Flüssigkeitsstrom der Einstauflüssigkeit 3 durch dünne gestrichelte Pfeile und die Gasabführung durch dünne durchgängige Pfeile dargestellt.

Der Fermenter 1 besitzt einen im unteren Bereich des Fermenters 1 angeordneten Feststoffeinlass 4. Die zu vergärenden organischen Feststoffe 2 werden aus einem Feststoffspeicher 5 über eine Fördereinrichtung 6, zum Beispiel eine Förderschnecke 6-1, in den Fermenter 1 eingebracht. Ebenfalls im unteren Bereich des Fermenters 1 ist der Flüssigkeitseinlass 7 angebracht, durch den die Einstauflüssigkeit 3 in den Fermenter 1 eingebracht wird.

Die Einstauflüssigkeit 3 zirkuliert durch den Fermenter 1, indem sie durch den Flüssigkeitseinlass 7 in den Fermenter 1 eingebracht wird und durch die Flüssigkeitsentnahme 8 wieder aus dem Fermenter 1 abgelassen wird. Durch diese Zirkulation entsteht eine aufwärtsgerichtete Strömung im Fermenter 1, durch die das Aufschwimmen der Feststoffe 2 unterstützt wird. Damit an der Flüssigkeitsentnahme 8 kein Fermentationsgas 16 austritt befindet sich an der Flüssigkeitsentnahme 8 ein Siphon 20.

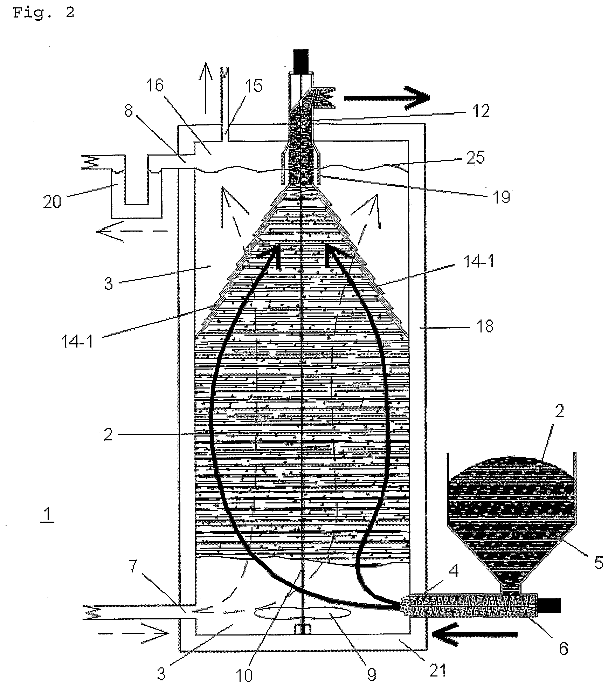

Figur 2 zeigt ebenfalls einen Querschnitt durch einen Fermenter 1 zur kontinuierlichen Verflüssigung von organischen Feststoffen 2. Bei dem in Figur 2 gezeigten Fermenter 1 ist jedoch zur Verbesserung der Verteilung neu zugeführter Feststoffe 2 ein Rührer 9 auf dem Fermenterboden angeordnet. Dieser Rührer 9 ist durch eine Welle 10 mit der Press- und Förderschnecke 11 in der Gärresteentnahme 12 gekoppelt.

Die Press- und Förderschnecke 11 dient der simultanen Entnahme und Entwässerung der festen Gärreste 13. Unter der Press- und Förderschnecke 11 ist ein Sieb 14 angebracht. Durch dieses lammellenförmige Spaltensieb 14-1 werden flüssige und gasförmige Bestandteile abgetrennt. Das Sieb 14 verjüngt sich trichterartig in Richtung zur Press- und Förderschnecke 11, so dass die Feststoffe 2 direkt der Press- und Förderschnecke 11 und somit der Gärresteentnahme 12 zugeführt werden.

An der Oberseite des Fermenters 1 befindet sich eine Gasentnahme 15, durch die das Fermentationsgas 16 aus dem Fermenter 1 herausgeführt wird.

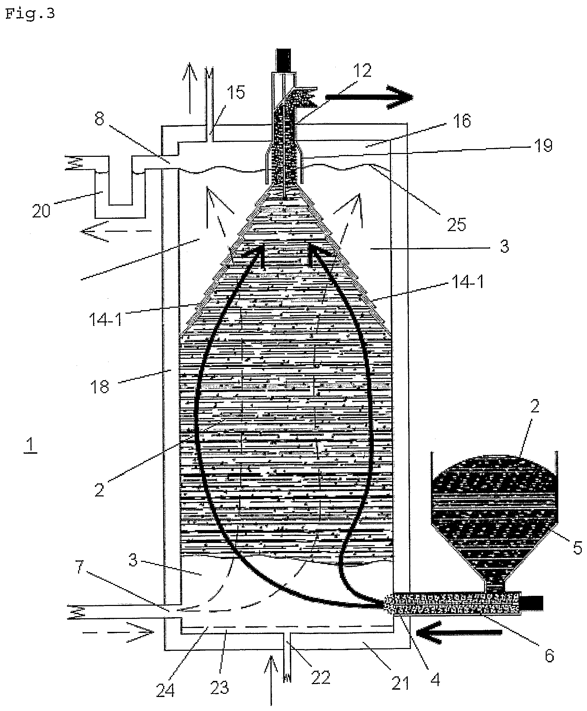

Figur 3 zeigt ein Querschnitt durch eine weitere Ausführungsform des Fermenters 1. Hierbei wird im unteren Bereich des Fermenters 1 eine Vergleichmäßigung der neu zugeführten Feststoffe 2 durch einen am Fermenterboden 21 angebrachten Gaseinlass 22 ermöglicht. Durch die Gaszuführung 22 wird dabei Gas durch Gasverteilerrohre 23 mit Öffnungen 24, die sich am Boden des Fermenters 1 befinden geblasen. Hierdurch werden außerdem am Fermenterboden 21 angesammelte Feststoffe 2 wieder mit der Einstauflüssigkeit 3 verwirbelt. Die Verwirbelung erfolgt dabei jedoch in einer Weise, dass das Aufschwimmen der Feststoffe nicht beeinträchtigt wird.

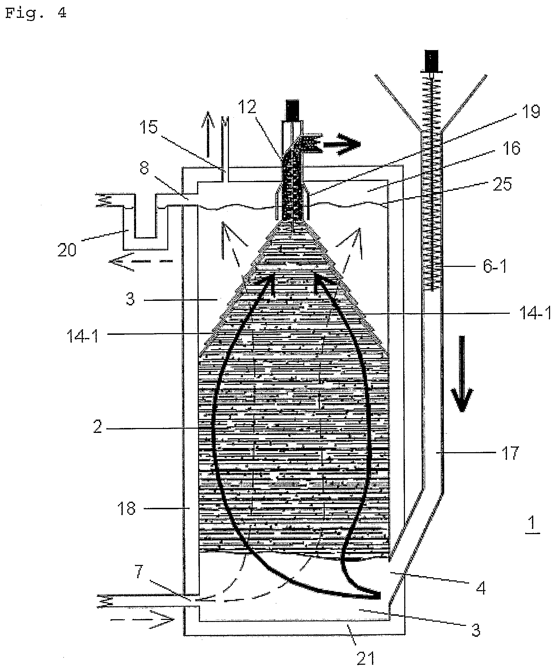

Die Figur 4 zeigt einen Fermenter 1 mit einer anderen Ausführungsform der Zuführung der Feststoffe 2. Bei der in der Figur 4 gezeigten Ausführungsform wird der Feststoff 2 durch ein außen liegendes Tauchrohr 17 in den Feststoffeinlass 4 des Fermenters 1 eingebracht.

Der Feststoffeinlass 4 ist dabei schräg nach oben abgewinkelt und nicht, wie in den Figuren 1 bis 3 gezeigten Ausführungsformen, horizontal zur Fermenterwand 18 angebracht.

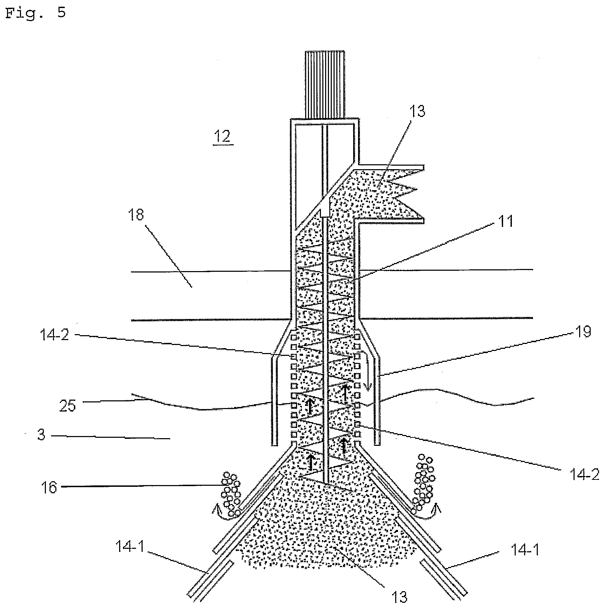

Die Figur 5 zeigt eine Detailansicht des Fermenters 1 im Querschnitt. Figur 5 verdeutlicht, wie das Fermentationsgas 16 durch die Spalten des lammelenförmigen Spaltensiebs 14-1 entweichen kann, während die festen Gärreste 13 durch die Press- und Förderschnecke 11 der Gärresteentnahme 12 zugeführt werden (dicke Pfeile: Feststoffstrom). Die Einstauflüssigkeit 3 kann sowohl durch die Spalten des lamellenförmigen Spaltsiebes entweichen (durch dünne Pfeile dargestellt), als auch durch ein Sieb 14-2 der Press- und Förderschnecke 11.

Zur gasdichten Abkapselung der Gärresteentnahme 12 dient ein in die Einstauflüssigkeit 3 getauchter Mantel 19. Die Öffnung 24 der Gärresteentnahme 12 befindet sich somit unterhalb des Flüssigkeitsniveaus 25.

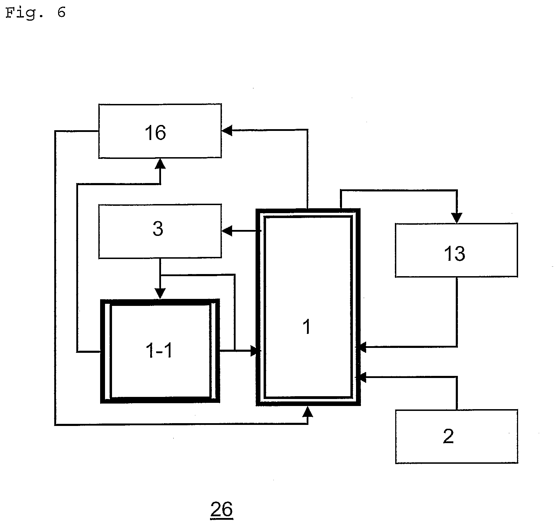

Des Weiteren zeigt Figur 6 ein Übersichtsschema einer Verflüssigungsanlage 26. In dem Fermenter 1 findet die Verflüssigung der Feststoffe 2 statt. Hierzu werden dem Fermenter 1 organische Feststoffe 2 und die Einstauflüssigkeit 3 zugeführt. Die Feststoffe 2 können dann aus dem Fermenter 1 als feste Gärreste 13 ausgebracht werden. Bei bedarf können die festen Gärreste 13 wieder in den Fermenter 1 zurückgeführt werden. Die Einstauflüssigkeit 3 und die im Fermenter 1 entstehenden Fermentationsgase 16 können ebenfalls wieder in den Fermenter 1 zurückgeführt werden. Es ist aber auch möglich, dass die Einstauflüssigkeit 3 in einen weiteren Flüssigkeits-Fermenter 1-1 gepumpt wird und von dort wieder in den Fermenter 1 gelangt oder aus dem Prozess herausgezogen wird.

Die Endprodukte des Verfahrens sind speicherbar.

### Bezugszeichenliste:

- 1: Fermenter
- 1-1: Flüssigkeits-Fermenter
- 2: Feststoffe
- 3: Einstauflüssigkeit
- 4: Feststoffeinlass
- 5: Feststoffspeicher
- 6: Fördereinrichtung
- 6-1: Förderschnecke
- 7: Flüssigkeitseinlass
- 8: Flüssigkeitsentnahme
- 9: Rührer
- 10: Welle
- 11: Press- und Förderschnecke
- 12: Gärresteentnahme
- 13: feste Gärreste
- 14: Sieb
- 14-1: lamellenförmiges Spaltensieb
- 14-2: Sieb der Press- und Förderschnecke
- 15: Gasentnahme
- 16: Fermentationsgas
- 17: Tauchrohr
- 18: Fermenterwand
- 19: Mantel
- 20: Siphon
- 21: Fermenterboden
- 22: Gaseinlass
- 23: Gasverteilerrohr
- 24: Öffnung
- 25: Flüssigkeitsniveau
- 26: Verflüssigungsanlage

## Patentansprüche

1. Verfahren zur kontinuierlichen Verflüssigung organischer Feststoffe (2) in einem Fermenter (1), **dadurch gekennzeichnet, dass** man
eine aufwärtsgerichtete Strömung der Feststoffe (2) in einer Einstauflüssigkeit (3) erzeugt, wobei man die Feststoffe (2) im unteren Bereich des Fermenters (1) zugibt,
die festen Gärreste (13) unterhalb des Einstauflüssigkeitsniveaus (25) mittels zumindest eines Siebes (14) im oberen Bereich des Fermenters (1) sammelt,
dadurch im Fermenter (1) eine Pfropfenströmung fester zu vergärender organischer Substanzen mit einem nach oben ansteigenden Dichtegradienten erzeugt,
die Einstauflüssigkeit (3) durch den Fermenter (1) zirkuliert, indem man sie durch den Flüssigkeitseinlass (7) im unteren Bereich des Fermenters (1) einbringt und durch die Flüssigkeitsentnahme (8) im oberen Bereich des Fermenters ablässt, wobei man durch die Zirkulation der Einstauflüssigkeit im Fermenter eine aufwärtsgerichtete Strömung erzeugt, die das Aufschwimmen der Feststoffe unterstützt,
die organischen Feststoffe für eine bestimmte Dauer in der Einstauflüssigkeit (3) verweilen lässt,
die festen Gärreste (13) anschließend im oberen Bereich des Fermenters (1) entnimmt,
die Fermentationsgase im oberen Bereich des Fermenters (1) entnimmt und
die gelösten organischen Substanzen entnimmt.

2. Verfahren nach Anspruch 1, **dadurch gekennzeichnet, dass** man unterhalb des Einstauflüssigkeitsniveaus (25)
die festen Gärreste (13) mit einer Press- und/oder Förderschnecke (11) entnimmt oder
mit einem Feststoffschaber in ein Fallrohr oder einen Schacht leitet oder
an der Position, an der die festen Gärreste (13) entnommen werden, einen Unterdruck erzeugt.

3. Verfahren nach Anspruch 1 oder 2, **dadurch gekennzeichnet, dass** man die Feststoffe (2) mit einer Fördereinrichtung (6) in den Fermenter (1) einbringt, welche eine Förderstrecke aufweist, die oberhalb des Flüssigkeitsniveaus (25) des Fermenters (1) beginnt.

4. Verfahren nach einem der Ansprüche 1 bis 3, **dadurch gekennzeichnet, dass** man die Feststoffe (2) im unteren Bereich des Fermenters (1) verteilt.

5. Verfahren nach einem der Ansprüche 1 bis 4, **dadurch gekennzeichnet, dass** man
Teile der Einstauflüssigkeit (3), Teile der festen Gärreste (13) und/oder des Fermentationsgases (16) durch den Fermenter (1) oder durch mehrere Fermenter zirkuliert und
die Einstauflüssigkeit (3) aus dem Fermenter (1) in einen zweiten Fermenter (1-1) überführt und von dort wieder zurück in den ersten Fermenter (1) führt oder
die Einstauflüssigkeit (3) und/oder die festen Gärreste (13) aus dem Fermenter (1) in einem zweiten Fermenter (1-1) überführt, wobei man in dem zweiten Fermenter (1-1) ein methan- und/oder wasserstoffhaltiges Fermentationsgas (16) erzeugt, das man bei Bedarf in den ersten Fermenter (1) zurückführt oder
die festen Gärreste (13) dem Fermenter (1) entnimmt und wieder in den Fermenter (1) überführt oder aus dem Fermenter (1) in einen zweiten Fermenter (1-1) leitet.

6. Verflüssigungsanlage (26) mit mindestens einem Fermenter (1) für ein Verfahren nach einem der vorhergehenden Ansprüche, **dadurch gekennzeichnet, dass** der Fermenter (1)
im oberen Bereich
mindestens eine Flüssigkeitsentnahme (8),
mindestens ein Sieb (14), das unterhalb des Einstauflüssigkeitsniveaus (25) angebracht ist, zum Sammeln der festen Gärreste im oberen Bereich des Fermenters,
jeweils mindestens eine Gärrestentnahme (12) und eine Gasentnahme (15)
sowie im unteren Bereich
jeweils mindestens einen Feststoffeinlass (4) und einen Flüssigkeitseinlass (7) enthält.

7. Verflüssigungsanlage (26) nach Anspruch 6, **dadurch gekennzeichnet, dass** der Fermenter (1) mindestens einen Gaseinlass (22) aufweist.

8. Verflüssigungsanlage (26) nach Anspruch 6 oder 7, **dadurch gekennzeichnet, dass** an dem Feststoffeinlass (4) des Fermenters (1) eine Fördereinrichtung (6) angebracht ist, wobei der Beginn der Förderstrecke oberhalb des Flüssigkeitsniveaus (25) des Fermenters (1) angeordnet ist.

9. Verflüssigungsanlage (26) nach einem der Ansprüche 6 oder 7, **dadurch gekennzeichnet, dass** an dem Feststoffeinlass (4) des Fermenters (1) eine Vorrichtung zur mechanischen, hydraulischen oder pneumatischen Förderung der Feststoffe (2) angeordnet ist.

10. Verflüssigungsanlage (26) nach einem der Ansprüche 6 bis 9, **dadurch gekennzeichnet, dass**
a)die Gärresteentnahme (12) des Fermenters (1)
i) eine Press- und/oder Förderschnecke (11) ist oder
ii) ein Fallrohr oder Fallschacht ist, wobei im unteren Bereich des Fallrohres oder Fallschachtes ein flüssigkeitsgefüllter Siphon oder eine Tauchung angebracht ist oder
iii) ein Fallrohr oder Fallschacht ist, wobei im unteren Bereich des Fallrohres oder Fallschachtes ein flüssigkeitsgefüllter Siphon oder eine Tauchung angebracht ist, und weiterhin einen rotierenden Feststoffschaber aufweist
oder
b) an der Gärresteentnahme (12) des Fermenters (1) ein Unterdruck erzeugbar ist.

11. Verflüssigungsanlage (26) nach einem der Ansprüche 6 bis 10, **dadurch gekennzeichnet, dass** auf dem Boden des Fermenters (1) ein Rührer (9) angebracht ist.

12. Verflüssigungsanlage (26) nach Anspruch 11, **dadurch gekennzeichnet, dass** die Press- und/oder Förderschnecke (11) der Gärresteentnahme (12) des Fermenters (1) und der Rührer (9) miteinander gekoppelt sind.

13. Verflüssigungsanlage (26) nach einem der Ansprüche 6 bis 12, **dadurch gekennzeichnet, dass** das Sieb (14) des Fermenters (1) trichterförmig ist.

14. Verflüssigungsanlage (26) nach einem der Ansprüche 6 bis 13, **dadurch gekennzeichnet, dass** das Sieb (14) aus schräg angeordneten Lamellen besteht.

15. Verwendung der Verflüssigungsanlage (26) nach einem der Ansprüche 6 bis 14 zur Herstellung
eines methan- und/oder wasserstoffhaltigen Fermentationsgases (16) oder
von organischen Säuren, die man in mindestens einem weiteren Fermenter zu einem methan- und/oder wasserstoffhaltigen Fermentationsgas abbaut oder
zur Entkeimung von Speiseresten.

## Claims

1. Method for the continuous liquefying of organic solids (2) in a fermenter (1), **characterized in that**
an upwardly directed flow of solids (2) is produced in a dammed-up liquid (3), wherein solids (2) are added in the lower region of the fermenter (1),
the solid fermentation residues (13) are collected below the level (25) of the dammed-up liquid by means of at least one sieve (14) in the upper region of the fermenter (1),
thereby a flow of droplets of solids to fermenting organic substances with an upwardly rising density gradient is produced in the fermenter (1),
the dammed-up liquid (3) circulates through the fermenter (1), by introducing it through the liquid inlet (7) in the lower region of the fermenter (1) and removing it through the liquid discharge outlet (8) in the upper region of the fermenter (1), whereby an upwardly directed flow in the fermenter (1) is produced by the circulation of the dammed-up liquid (3), which supports the floating-up of the solids,
the organic solids reside for a specific time in the dammed-up liquid (3),
the solid fermentation residues (13) are subsequently removed in the upper region of the fermenter (1),
the fermentation gases (16) are removed in the upper region of the fermenter (1) and
the solved organic substances are removed.

2. Method according to claim 1, **characterized in that** the solid fermentation residues (13) are removed below the level (25) of the dammed-up liquid by a pressure and/or feed screw conveyor (11) or
are guided by a solids scraper into a downpipe or a shaft or
that an underpressure is produced at the position where the solid fermentation residues (13) are removed.

3. Method according to claim 1 or 2, **characterized in that** the solids (2) are introduced into the fermenter (1) by a conveyor device (6), which has a conveyance path that begins above the liquid level (25) of the fermenter (1).

4. Method according to one of claims 1 to 3, **characterized in that** the solids (2) are spread out in the lower region of the fermenter (1).

5. Method according to one of claims 1 to 4, **characterized in that**
parts of the dammed-up liquid (3), parts of the solid fermentation residues (13) and/or fermentation gas (16) are circulated through the fermenter (1) or through several fermenters and
the dammed-up liquid (3) is transferred from the fermenter into a second fermenter (1-1) and from there back again into the first fermenter (1) or
the dammed-up liquid (3) and/or the solid fermentation residues (13) are transferred from the fermenter (1) into a second fermenter (1-1), whereby a fermentation gas (16), which contains methane and/or hydrogen, is produced in the second fermenter (1-1), which is returned to the first fermenter (1) as needed, or
the solid fermentation residues (13) are removed from fermenter (1) and are again transferred back to the fermenter (1) or are guided to a second fermenter (1-1) from the fermenter (1).

6. Liquefying plant (26) with at least one fermenter (1) for a method according to one of the preceding claims, **characterized in that** the fermenter (1) contains
in the upper region
at least one liquid discharge outlet (8),
at least one sieve (14), which is positioned below the level (25) of the dammed-up liquid to collect the solid fermentation residues (13) in the upper region of the fermenter (1),
respectively at least one discharge outlet for fermentation residues (12) and one discharge outlet for gas (15)
as well as in the lower region
respectively at least one solids inlet (4) and one liquid inlet (7).

7. Liquefying plant (26) according to claim 6, **characterized in that** the fermenter (1) has at least one gas inlet (22).

8. Liquefying plant (26) according to claim 6 or 7, **characterized in that** a conveyor device (6) is accommodated at the solids inlet (4) of the fermenter (1), whereby the beginning of the conveyance path is disposed above the liquid level (25) of the fermenter (1).

9. Liquefying plant (26) according to one of the clams 6 or 7, **characterized in that** a device for the mechanical, hydraulic or pneumatic conveyance of solids (2) is disposed at the solids inlet (4) of the fermenter (1).

10. Liquefying plant (26) according to one of the claims 6 to 9, **characterized in that**
a)the discharge outlet (12) for the fermentation residues of the fermenter (1)
i) is a pressure and/or feed screw conveyor (11) or
ii) a downpipe or down shaft, whereby a liquid-filled siphon or an immersed device is introduced in the lower region of the downpipe or down shaft or
iii) a downpipe or down shaft, whereby a liquid-filled siphon or an immersed device is introduced in the lower region of the downpipe or down shaft and further provides a rotating solid scraper
or
b) an underpressure can be produced at the discharge outlet (12) for the fermentation residues of the fermenter (1).

11. Liquefying plant (26) according to one of the claims 6 to 10, **characterized in that** a stirrer (9) is accommodated at the bottom of fermenter (1).

12. Liquefying plant (26) according to claim 11, **characterized in that** the pressure and/or feed screw conveyor (11) of the discharge outlet (12) for the fermentation residues of the fermenter (1) and the stirrer (9) are coupled with one another.

13. Liquefying plant (26) according to one of the claims 6 to 12, **characterized in that** the sieve (14) of the fermenter (1) is funnel-shaped.

14. Liquefying plant (26) according to one of the claims 6 to 13, **characterized in that** the sieve (14) consists of obliquely disposed lamellae.

15. Use of the liquefying plant (26) according to one of the claims 6 to 14 for the production of
a fermentation gas (16) containing methane and/or hydrogen or
of organic acids, which are decomposed in at least one other fermenter to form a fermentation gas containing methane and/or hydrogen or
for eliminating germs from food wastes.

## Revendications

1. Procédé pour liquéfier en continu des substances organiques solides (2) dans un fermenteur (1), **caractérisé en ce que**
l'on produit un flux ascendant de substances solides (2) dans un liquide en milieu fermé (3), auquel cas les substances solides (2) sont ajoutées dans la partie inférieure du fermenteur (1),
l'on rassemble dans la partie supérieure du fermenteur (1) les résidus de fermentation solides (13) qui se trouvent en-dessous du niveau du liquide en milieu fermé (25) à l'aide d'au moins un tamis (14),
l'on produit ainsi à l'intérieur du fermenteur (1) un flux à effet bouchon des substances organiques solides à fermenter avec un gradient de densité croissant,
le liquide en milieu fermé (3) circule à travers le fermenteur (1), en l'introduisant à travers l'admission de fluide (7) dans la partie inférieure du fermenteur (1) et en l'évacuant à travers le prélèvement de liquide (8) dans la partie supérieure du fermenteur, auquel cas l'on produit par la circulation du liquide en milieu fermé dans le fermenteur un flux ascendant qui soutient la flottabilité des substances solides,
l'on laisse séjourner les substances organiques solides pendant un laps de temps défini dans le liquide en milieu fermé (3),
l'on prélève les résidus de fermentation solides (13) par la suite dans la partie supérieure du fermenteur (1),
l'on prélève les gaz de fermentation dans la partie supérieure du fermenteur (1) et
l'on prélève les substances organiques dissoutes.

2. Procédé selon la revendication 1, **caractérisé en ce que** en-dessous du niveau du liquide en milieu fermé (25)
l'on prélève les résidus de fermentation solides (13) avec un pressoir à vis sans fin et/ou une vis transporteuse (11) ou
on les conduit avec un racleur en matériau solide dans un tuyau de descente ou dans un puits de chute ou
l'on produit une dépression au niveau de la position dans laquelle les résidus de fermentation solides (13) sont prélevés.

3. Procédé selon la revendication 1 ou 2, **caractérisé en ce que** l'on introduit dans le fermenteur (1) les substances solides (2) avec un dispositif convoyeur (6) qui présente un chemin de transport dont le début se situe au-dessus du niveau du liquide (25) du fermenteur (1).

4. Procédé selon l'une quelconque des revendications 1 à 3, **caractérisé en ce que** l'on distribue les substances solides (2) dans la partie inférieure du fermenteur (1).

5. Procédé selon l'une quelconque des revendications 1 à 4, **caractérisé en ce que**
des parties du liquide en milieu fermé (3), des parties des résidus de fermentation solides (13) et/ou du gaz de fermentation (16) circulent à travers le fermenteur (1) ou à travers plusieurs fermenteurs et
l'on transfère le liquide en milieu fermé (3) hors du fermenteur (1) dans un deuxième fermenteur (1-1) et de là on le retransfère à nouveau dans le premier fermenteur (1) ou
l'on transfère le liquide en milieu fermé (3) et/ou les résidus de fermentation solides (13) hors du fermenteur (1) dans un deuxième fermenteur (1-1), auquel cas l'on produit dans le deuxième fermenteur (1-1) un gaz de fermentation contenant de l'hydrogène et/ou du méthane (16) que l'on peut ramener, au besoin, dans le premier fermenteur (1) ou
l'on prélève les résidus de fermentation solides (13) hors du fermenteur (1) et on les transfère à nouveau dans le fermenteur (1) ou on les conduit du fermenteur (1) dans un deuxième fermenteur (1-1).

6. Installation de liquéfaction (26) dotée d'au moins un fermenteur (1) pour un procédé selon l'une quelconque des revendications précédentes, **caractérisée en ce que** le fermenteur (1) comporte
dans la partie supérieure
au moins un prélèvement de liquide (8),
au moins un tamis (14) qui est monté en-dessous du niveau du liquide en milieu fermé (25) pour collecter les résidus de fermentation solides dans la partie supérieure du fermenteur (1),
respectivement au moins un prélèvement de résidus de fermentation (12) et un prélèvement de gaz (15)
ainsi que dans la partie inférieure
respectivement au moins une admission de substances solides (4) et une admission de liquides (7).

7. Installation de liquéfaction (26) selon la revendication 6, **caractérisée en ce que** le fermenteur (1) présente au moins une admission de gaz (22).

8. Installation de liquéfaction (26) selon la revendication 6 ou 7, **caractérisée en ce que** l'on monte sur l'admission de substances solides (4) du fermenteur (1) un dispositif convoyeur (6), auquel cas le début du chemin de transport se situe au-dessus du niveau du liquide (25) du fermenteur (1).

9. Installation de liquéfaction (26) selon l'une quelconque des revendications 6 ou 7, **caractérisée en ce que** un dispositif pour le transport mécanique, hydraulique ou pneumatique des substances solides (2) est agencé sur l'admission de substances solides (4) du fermenteur (1).

10. Installation de liquéfaction (26) selon l'une quelconque des revendications 6 à 9, **caractérisée en ce que**
a) le prélèvement des résidus de fermentation (12) du fermenteur (1)
i) est un pressoir à vis sans fin et/ou une vis transporteuse (11) ou
ii) est un tuyau de descente ou un puits de chute, auquel cas un siphon rempli de liquide ou un dispositif d'immersion est monté dans la partie inférieure du tuyau de descente ou du puits de chute ou
iii) est un tuyau de descente ou un puits de chute, auquel cas un siphon rempli de liquide ou un dispositif d'immersion est monté dans la partie inférieure du tuyau de descente ou du puits de chute et présente, en outre, un racleur rotatif en matériau solide ou
b) l'on peut produire une dépression au niveau du prélèvement des résidus de fermentation (12) du fermenteur (1).

11. Installation de liquéfaction (26) selon l'une quelconque des revendications 6 à 10, **caractérisée en ce que** l'on monte sur le fond du fermenteur (1) un agitateur (9).

12. Installation de liquéfaction (26) selon la revendication 11, **caractérisée en ce que** le pressoir à vis sans fin et/ou la vis transporteuse (11) du prélèvement de résidus de fermentation (12) du fermenteur (1) et l'agitateur (9) sont couplés l'un à l'autre.

13. Installation de liquéfaction (26) selon l'une quelconque des revendications 6 à 12, **caractérisée en ce que** le tamis (14) du fermenteur (1) est en forme d'entonnoir.

14. Installation de liquéfaction (26) selon l'une quelconque des revendications 6 à 13, **caractérisée en ce que** le tamis (14) se compose de lamelles agencées obliquement.

15. Utilisation de l'installation de liquéfaction (26) selon l'une quelconque des revendications 6 à 14 pour la production
d'un gaz de fermentation contenant de l'hydrogène et/ou du méthane (16)
ou
d'acides organiques que l'on dégrade dans au moins un autre fermenteur en un gaz de fermentation contenant de l'hydrogène et/ou du méthane ou
pour la décontamination de déchets alimentaires.
